# EUROPÄISCHE PATENTANMELDUNG

(11) **EP 4 279 060 A1**
(43) Veröffentlichungstag der Anmeldung: **22.11.2023**
(21) Anmeldenummer: 23167446.6
(22) Anmeldetag: 11.04.2023
(51) Int. Cl.: A61K 8/73, A61Q 5/06, A61Q 17/04

(54) **HAAR-STYLING-ZUBEREITUNGEN, DIE EINEN UV-SCHUTZ BEWIRKEN**

(30) Priorität: 16.05.2022 DE 102022204781
(71) Anmelder: Beiersdorf Aktiengesellschaft, 20245 Hamburg (DE)
(72) Erfinder: Budnick, Nina, 22147 Hamburg (DE); Stoltze, Pia, 22455 Hamburg (DE); Nemnich, Julia, 22589 Hamburg (DE); Hetzel, Frank, 21261 Welle (DE)
(74) Vertreter: Beiersdorf AG

(57) **Zusammenfassung**

Verwendung einer Kombination von Pullulan und einer modifizierten Stärkeverbindung in einer Haar-Styling-Zubereitung zum UV-Schutz der Haare.

## Beschreibung

Die vorliegende Erfindung beschreibt kosmetische Haar-Styling-Zubereitungen in Form eines Schaumfestigers, eines Haargels oder einer Styling-Emulsion zum Stylen von menschlichen Haaren, die ein natürliches Styling-Polymer enthalten. Durch die Anwendung der genannten Haar-Styling-Zubereitungen erhalten die Haare einen UV-Schutz.

Haare sind ein wichtiger Teil des menschlichen Körpers. Der ganze menschliche Körper mit Ausnahme der Lippen, der Handinnenflächen und der Fußsohlen, ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft ist aus drei Schichten aufgebaut: Der zentrale Teil wird als Haarmark oder Medulla bezeichnet. Dieser Teil ist allerdings beim Menschen zurückgebildet und fehlt oft. An den zentralen Teil schließt sich die Haarrinde oder Cortex an. Diese Faserschicht besteht aus verhornten Faserzellen und bestimmt die Festigkeit und Elastizität des Haares. In dieser Schicht sind auch die Farbpigmente enthalten. Außen um die Faserschicht ist die Schuppenschicht oder Cuticula angeordnet. Diese Schicht ist mehrlagig, sehr dünn und durchsichtig.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf.

Zu den äußeren Einflüssen, die auf das Haar einwirken, ist auch UV-Licht zu zählen. UV-Licht ist ein Energie-reiches Licht, das zum einen auf Farbpigmente einwirkt und sie zerstören kann. Dies gilt für die natürlichen Farbpigmente, die Melanine, genauso wie für Pigmente oder Farbstoffe, die mittels Haarfärbungen oder Haartönungen auf das Haar gelangen. Durch das UV-Licht wird die Farbe der Haare blasser, die Haare bleichen aus.

Zum anderen wirkt das UV-Licht auch auf die Proteinstrukturen der Haare ein und schädigt diese. Die Folge sind Haare mit Spliss und/oder Haare, die brüchig sind.

Darüber hinaus führt das UV-Licht auch dazu, dass die Haare ein stumpfes Aussehen bekommen.

Es ist also überaus wünschenswert, für die Haare einen UV-Schutz bereit zu stellen, um den genannten negativen Phänomenen entgegenzuwirken.

Natürliches Haar hat in vielen Fällen wenig eigenes Volumen und bei langem Haar hängt es oftmals recht schlaff und gerade vom Kopf herab. Viele Menschen wünschen sich aber Haare, die mehr Volumen aufweisen und sich auf gefällige Art formen (stylen) lassen. Dies kann grundsätzlich auf zweierlei Weise erzielt werden, zum einen gibt es permanente Haarverformungsverfahren, beispielsweise die Dauerwelle und nicht permanente Verfahren, die die Frisur des Haares nur für eine begrenzte Zeit formen und fixieren. Um eine nicht-permanente Frisurgestaltung zu erreichen, werden mehrere Produktformen zur Verfügung gestellt, die einzeln oder in Kombination miteinander verwendet werden können. Zu nennen seien hier beispielsweise, Festiger, Schaumfestiger, Haarsprays, Haarwachse, Haargele, Styling-Emulsionen und andere mehr.

Schaumfestiger sind beim Verbraucher sehr beliebt. Sie werden in der Regel auf das nasse, handtuchtrockene oder trockene Haar aufgetragen. Anschließend wird das Haar zu einer Frisur geformt (gestylt), oft mithilfe eines Föns. Die Schaumfestiger zeichnen sich dadurch aus, dass sie Polymere enthalten, die eine Fixierung der Frisur bewirken und so zum Frisurerhalt geeignet sind. Zumeist werden derartige Produkte nach jeder Haarwäsche angewandt. Ebenso beliebt sind auch andere Stylingzubereitungen, beispielsweise in Form von Haargelen und Styling-Emulsionen.

Wünschenswert ist nun eine Haar-Styling-Zubereitung, die die Haare pflegt, stylt und/oder formt und gleichzeitig einen Schutz vor UV-Strahlen bewirkt.

Im StdT finden sich Dokumente, die offenbaren, dass ein UV-Schutz für die Haare bewirkt werden kann, indem bekannte UV-Filter den Zubereitungen für die Haare zugesetzt werden. Beispielhaft seien die Dokumente WO 2011/009815 A2 und EP 2606934 A2 genannt. Auch in Produkten der Firma Beiersdorf wird eine UV-absorbierende Substanz, nämlich Benzophenone-4, eingesetzt. Beispielhaft seien die Produkte Styling Mousse Diamant Volumen (Mintel Eintragsnummer 5350263), Styling Mousse Colorschutz (Mintel Eintragsnummer 5323925) und Styling Mousse Volumen Pflege (Mintel Eintragsnummer 5233205) genannt.

Da die genannten, bekannten UV-Filter weniger gut in Zubereitungen für die Haare eingearbeitet werden können und/oder auf den Haaren nicht gut haften bleiben, wurden ausgewählte Polymere zum UV-Schutz verwendet, um die genannten Nachteile zu überwinden. Dies wird in den Dokumenten WO 2005/025491 A2, WO 2005/044948 A2 und WO 2010/078311 A1 beschrieben.

Aus keinem der genannten Dokumente konnte der Fachmann jedoch Hinweise entnehmen, dass auch andere Polymere diesen UV-Schutz bewirken können.

Zudem sollte den Verbraucherwünschen nach Zubereitungen, die nur oder doch weitgehend natürliche Inhaltsstoffe enthalten, die zudem gut biologisch abbaubar sind Rechnung getragen werden. Dies bedeutet u.a. ein Verzicht auf bekannte festigende und/oder Film-bildende Polymere, die in der Regel synthetischer Natur sind und in der Folge meistens auch nicht gut biologisch abbaubar sind.

Überraschenderweise wurde gefunden, dass Pullulan in Verbindung mit wenigstens einer modifizierten Stärkeverbindungen und/oder wenigstens einer prägelatinisierten Stärke einen UV-Schutz für die Haare bewirkt.

Die Einarbeitung von Pullulan in Haar-kosmetische Zubereitungen ist in den (noch nicht veröffentlichten) Anmeldungen DE 102021206452.3, DE 10201206451.5 und DE 102021206450.7 offenbart. Die Anmeldung DE 102021206450.7 ist auf Schaumfestigerzubereitungen gerichtet, die neben Pullulan auch eine modifizierte Stärkeverbindung enthalten. In keinem der Dokumente ist offenbart, dass die Kombination Pullulan und eine modifizierte Stärkeverbindung und/oder eine prägelatinisierte Stärke Vorteile in Bezug auf einen UV-Schutz haben könnte.

Somit ist der Gegenstand der vorliegenden Anmeldung die Verwendung der Kombination von Pullulan und wenigstens einer modifizierten Stärkeverbindung und/oder wenigstens einer prägelatinisierten Stärke in einer Haar-Styling-Zubereitung zum UV-Schutz der Haare, insbesondere der menschlichen Kopfhaare. Es ist vorteilhaft, wenn die Kombination aus Pullulan und die wenigstens einer modifizierter Stärkeverbindung besteht. Es ist weiter vorteilhaft, wenn Pullulan und die wenigstens eine modifizierte Stärkeverbindung in einem bestimmten Gewichtsverhältnis vorliegen, und zwar im Gewichtsverhältnis von Pullulan zu der wenigstens einen Stärkeverbindung von 1,5:2 bis 1:5, vorzugsweise von 1:2 bis 1:3.

Ebenso Gegenstand der vorliegenden Erfindung ist die Verwendung eines Schaumfestigers, enthaltend die Kombination Pullulan und wenigstens eine modifizierte Stärkeverbindung und/oder wenigstens eine prägelatinisierte Stärke zum UV-Schutz der Haare, insbesondere der menschlichen Kopfhaare. Bevorzugt ist die Kombination Pullulan und wenigstens eine modifizierte Stärkeverbindung. Ausgehend von dieser bevorzugten Ausführungsform ist es weiter vorteilhaft, wenn zusätzlich wenigstens ein Cellulosemischether enthalten ist. Genauso weiter vorteilhaft ist es, wenn zusätzlich wenigstens ein nichtionisches Tensid, insbesondere wenigstens ein Alkylpolyglucosid, enthalten ist. Noch vorteilhafter ist es, wenn zusätzlich zu der Kombination von Pullulan und wenigstens eine modifizierte Stärkeverbindung wenigstens ein Cellulosemischether und wenigstens ein nichtionisches Tensid, insbesondere wenigstens ein Alkylpolyglucosid, enthalten ist.

Auch Gegenstand der vorliegenden Erfindung ist die Verwendung eines Haargels, enthaltend die Kombination Pullulan und wenigstens eine modifizierte Stärkeverbindung und/oder wenigstens eine prägelatinisierte Stärke zum UV-Schutz der Haare, insbesondere der menschlichen Kopfhaare. Bevorzugt ist die Kombination Pullulan und wenigstens eine modifizierte Stärkeverbindung. Ausgehend von dieser bevorzugten Ausführungsform ist es weiter vorteilhaft, wenn zusätzlich wenigstens ein Cellulosemischether enthalten ist. Genauso weiter vorteilhaft ist es, wenn zusätzlich wenigstens ein nichtionisches Tensid, insbesondere wenigstens ein Alkylpolyglucosid, enthalten ist. Noch vorteilhafter ist es, wenn zusätzlich zu der Kombination von Pullulan und wenigstens eine modifizierte Stärkeverbindung wenigstens ein Cellulosemischether und wenigstens ein nichtionisches Tensid, insbesondere wenigstens ein Alkylpolyglucosid, enthalten ist.

Weiterhin Gegenstand der vorliegenden Erfindung ist die Verwendung einer Styling-Emulsion, enthaltend die Kombination Pullulan und wenigstens eine modifizierte Stärkeverbindung und/oder wenigstens eine prägelatinisierten Stärke zum UV-Schutz der Haare, insbesondere der menschlichen Kopfhaare. Bevorzugt ist die Kombination Pullulan und wenigstens eine modifizierte Stärkeverbindung. Ausgehend von dieser bevorzugten Ausführungsform ist es weiter vorteilhaft, wenn zusätzlich wenigstens ein Cellulosemischether enthalten ist. Genauso weiter vorteilhaft ist es, wenn zusätzlich wenigstens ein nichtionisches Tensid, insbesondere wenigstens ein Alkylpolyglucosid, enthalten ist. Noch vorteilhafter ist es, wenn zusätzlich zu der Kombination von Pullulan und wenigstens eine modifizierte Stärkeverbindung wenigstens ein Cellulosemischether und wenigstens ein nichtionisches Tensid, insbesondere wenigstens ein Alkylpolyglucosid, enthalten ist.

Der Begriff Haare im Sinne der vorliegenden Erfindung bezieht sich auf menschliche Haare.

Im Sinne der vorliegenden Erfindung umfassen Haar-Styling-Zubereitungen alle Zubereitungen, die zum Stylen und/oder Formen der menschlichen Haare geeignet. Erfindungsgemäß vorteilhaft umfassen die Haar-Styling-Zubereitungen Schaumfestiger, Haargele und Styling-Emulsionen.

Der Begriff "natürlich" im Zusammenhang mit natürlichen Inhaltsstoffen für Kosmetika ist in der ISO 16128 festgelegt. Gemäß dieser Richtlinie kann man natürliche, kosmetische Inhaltsstoffe zusammenfassend als Stoffe beschreiben, die von Pflanzen, Tieren, Mineralien oder Mikroorganismen erhältlich sind, eingeschlossen sind auch solche Stoffe, die von den genannten Quellen durch physikalische Prozesse, Fermentationsprozesse (nur solche Fermentationsprozesse, die auch in der Natur ablaufen und die zu Produkten führen, die auch in der Natur entstehen) und andere Herstellungsmethoden ohne beabsichtigte chemische Modifikation erhalten werden. Die Mikroorganismen dürfen nur solche sein, die nicht gentechnisch modifiziert wurden.

Der Begriff der biologischen Abbaubarkeit beschreibt den Prozess des Abbaus von organischen Verbindungen durch Lebewesen, insbesondere Saprobionten. Im Idealfall entstehen anorganische Stoffe, wie beispielsweise CO₂, O₂ und Ammoniak; Verbindungen, die von Pflanzen und Mikroorganismen wieder für den Aufbau von organischen Verbindungen genutzt werden.

Organische, chemische Verbindungen, die leicht biologisch abbaubar sind, sind nach OECD 301, für kosmetische Zusammensetzungen meist nach OECD 301 B klassifiziert. Diese Substanzen oder Zusammensetzungen können rasch und vollständig abgebaut werden.

Organische, chemische Verbindungen, die nach OECD 302 klassifiziert sind, sind zwar eingeschränkt, jedoch grundsätzlich biologisch abbaubar.

Die Begriffe Haarschaumfestiger, Stylingschaum, Styling-Mousse und Schaumfestiger werden im Zusammenhang mit dieser Anmeldung synonym verwendet.

Der Wassergehalt der erfindungsgemäßen Zubereitungen beträgt vorteilhaft wenigstens 50 Gew.-%, bevorzugt wenigstens 55 Gew.-%, insbesondere bevorzugt 60 Gew.-%, bezogen auf das Gesamtgewicht der Zubereitung. Der Wassergehalt der erfindungsgemäßen Zubereitungen beträgt vorteilhaft höchstens 98 Gew.-%, bezogen auf das Gesamtgewicht der Zubereitung.

Pullulan ist ein wasserlösliches, lineares Polymer mit festigender/Film-bildender Wirkung. Es ist ein α-1,6 verknüpftes Polysaccharid aus Maltotriose-Einheiten, das aus dem Pilz *Aureobasidium pullulans* gewonnen werden kann. Die Glucose-Einheiten der Maltotriose sind α-1,4-glycosidisch miteinander verknüpft. Pullulan ist gut in Wasser löslich.

Pullulan kann beispielsweise unter dem Handelsnamen Pullulan von der Firma Hayashibara Co., LTD. bezogen werden.

In der erfindungsgemäßen Haar-Styling-Zubereitung ist Pullulan mit einem Gehalt von 0,1 bis 15,0 Gew.-%, bevorzugt von 0,5 bis 5,0 Gew.-% enthalten, bezogen auf das Gesamtgewicht der Haar-Styling-Zubereitung.

Die erfindungsgemäßen Haar-Styling-Zubereitungen enthalten wenigstens eine weitere Film-bildende Verbindung, nämlich eine modifizierte Stärkeverbindung. Die Stärkeverbindungen können aus verschiedenen Quellen stammen, beispielsweise Reisstärke, Maisstärke oder Kartoffelstärke. Die Gruppe der modifizierten Stärkeverbindungen hat auch verdickende Eigenschaften. Im Zusammenhang mit der vorliegenden Erfindung sind jedoch die Film-bildenden Eigenschaften relevant und tragen zur Erfindung bei. Die erfindungsgemäßen Stärkeverbindungen können nichtionische Biopolymere sein oder einen ionischen Charakter haben. Stärke ist ein Speicherkohlenhydrat der Pflanzen, das aus zwei Molekültypen besteht, Amylose und Amylopektin. Kennzeichnend für Amylose sind lineare helikale Ketten aus α-1,4-glykosidisch verknüpften Glukosemolekülen, während Amylopektin eine verzweigte Struktur aufweist. Diese Verzweigung wird durch zusätzliche α-1,6 glykosidisch verknüpfte Glukosemoleküle erreicht.

Modifizierte Stärken können durch physikalische, enzymatische oder chemische Verfahren ausgehend von nativer Stärke gewonnen werden. Durch diese Modifikationen können die Anwendungseigenschaften der Stärken verändert werden, um speziellen Anforderungen gerecht zu werden. Es gibt verschiedene Arten der Modifikation, von denen eine Auswahl hier genannt wird:
- Säurebehandelte Stärke durch Reaktion mit Säuren,
- Alkalisch modifizierte Stärke durch Reaktion mit Laugen,
- Gebleichte Stärke durch Behandlung mit Peroxyessigsäure, Wasserstoffperoxid, Natriumhypochlorit, Natriumchlorit, Schwefeldioxid, Sulfiten, Kaliumpermanganat oder Ammoniumpersulfat,
- Enzymatisch modifizierte Stärke durch Behandlung mit Amylasen
- Oxidierte Stärke durch Oxidation (z. B. mit Natriumhypochlorit)
- Monostärkephosphat durch Veresterung mit phosphorigen Estergruppen (z. B. Phosphorsäure, Natrium- oder Kaliumphosphat, Phosphonsäure oder Pentanatriumtriphosphat)
- Distärkephosphat durch Veresterung mit Natriumtrimetaphosphat oder Phosphoroxychlorid
- Phosphatiertes Distärkephosphat durch Kombination der Verfahren zur Herstellung von Monostärkephosphat und Distärkephosphat
- Stärkeacetat bzw. acetylierte Stärke durch Reaktion mit Essigsäureanhydrid oder Veresterung mit Essigsäure
- Hydroxypropylstärke durch Reaktion mit Propylenoxid.
- Stärkenatriumoctenylsuccinat durch Reaktion von Stärke mit Octenylbernsteinsäureanhydrid.

Erfindungsgemäß bevorzugt ist der Einsatz wenigstens einer modifizierten Maisstärkeverbindung.

Die Begriffe Stärkeverbindung und Stärke werden im Zusammenhang mit dieser Erfindung synonym verwendet, ebenso die Begriffe Maisstärkeverbindung und Maisstärke.

Modifizierte Maisstärke kann beispielsweise als Hydroxypropylstärke vorliegen, die INCI-Bezeichnung lautet Corn Starch Modified. Eine derartige Verbindung ist beispielsweise bei der Firma Nouryon unter der Handelsbezeichnung Amaze erhältlich. Weiterhin kann die Stärke auch als hydrolysierte Stärke vorliegen, dabei wird die Stärke meist durch eine Säurebehandlung, aber auch enzymatisch, zu Zwischenprodukten abgebaut. Derartige Produkte haben die INCI-Bezeichnung Hydrolyzed Corn Starch (erhältlich beispielsweise als MaizeCare Style Polymer bei der Firma Dow) oder Maltodextrin (erhältlich beispielsweise als Agenanova 30.326 von der Firma Agrana). Darüber hinaus kann die modifizierte Maisstärke auch als Distärkephosphat vorliegen, erhältlich beispielsweise als Agenajel 20.306 von der Firma Agrana. Diese Verbindung hat die INCI-Bezeichnung Distarch Phosphate. Eine Kombination aus hydroxypropylierter Stärke mit phosphatierter Stärke hat die INCI-Bezeichnung Hydroxyproyl Starch Phosphate und ist beispielsweise unter Bezeichnung Agenajel 20.383 Bei der Firma Agrana erhältlich.

Modifizierte Kartoffelstärke kann beispielsweise als hydroxypropylierte Stärke vorliegen; diese Verbindung hat die INCI-Bezeichnung Hydroxypropyl Starch und ist beispielsweise erhältlich bei der Firma Agrana unter der Handelsbezeichnung Amitrolit 8850.

Modifizierte Reisstärke kann beispielsweise als Distärkephosphat vorliegen; diese verbindung hat die INCI-Bezeichnung Distarch Phosphate und ist beispielsweise mit der Handelsbezeichnung Rice PO4 Natural bei der Firma Agrana erhältlich. Bevorzugt ist der Einsatz von Distarch Phosphate.

In der erfindungsgemäßen Haar-Styling-Zubereitung liegt die wenigstens eine modifizierte Stärkeverbindung mit einem Gesamtgehalt von 0,1 bis 8,0 Gew. %, bevorzugt von 0,5 bis 5,0 Gew. % vor, bezogen auf das Gesamtgewicht der Haar-Styling-Zubereitung.

Prägelatinisierte Stärke, auch gelatinisierte Stärke genannt, ist eine natürliche Stärke pflanzlichen Ursprungs, die beispielsweise aus Kartoffeln, Reis, Weizen, Mais, Bohnen und andern Pflanzen gewonnen wird und dann durch Erhitzen in Wasser, gegebenenfalls unter Zugabe von wenig Säure oder Alkali, weiter behandelt wird. Diese prägelatinisierten Stärken besitzen ein reduziertes Molekulargewicht und sind, infolge einer verringerten Viskosität, sehr gut zu verarbeiten. Prägelatinisierte Stärke ist beispielsweise unter dem Handelsnamen Agenajel 20.306 von der Firma Agrana zu erhalten.

In der erfindungsgemäßen Haar-Styling-Zubereitung liegt die wenigstens eine prägelatinisierte Stärkeverbindung mit einem Gesamtgehalt von 0,1 bis 8,0 Gew. %, bevorzugt von 0,5 bis 5,0 Gew. % vor, bezogen auf das Gesamtgewicht der Haar-Styling-Zubereitung.

Der erfindungsgemäßen Haar-Styling-Zubereitungen können optional wenigstens noch eine weitere Film- bildende Verbindung ausgewählt aus kationisch modifizierten Guar-Verbindungen enthalten. Dazu wird beispielsweise Guarkernmehl zunächst mit Propylenoxid verethert und anschließend weiter modifiziert zu Guar Hydroxypropyltrimonium Chlorid. Guar Hydroxypropyltrimonium Chlorid hat neben seiner Film-bildenden Wirkung auch einen antistatischen, Viskositäts-regulierenden sowie Haut- und Haar-pflegenden Effekt.

Guar Hydroxypropyltrimonium Chlorid ist mit verschiedenen Molekulargewichten und mit verschiedenen Ladungsdichten erhältlich. Als vorteilhaft haben sich Guar Hydroxypropyltrimonium Chlorid-Verbindungen mit einem Molekulargewicht von etwa 1,0 bis 3,0 Million Dalton erwiesen. Ebenso haben sich Guar Hydroxypropyltrimonium Chlorid-Verbindungen mit einer Ladungsdichte von 0,5 bis 1,0 mmol/g als vorteilhaft erwiesen.

Die Bestimmung des Molekulargewichts kann vorteilhaft mit der Methode der Gel-Permeations-Chromatographie bestimmt werden.

Die Ladungsdichte eines Polymers und in Bezug auf Guar Hydroxypropyltrimonium Chlorid-Verbindungen die kationische Ladungsdichte, bezieht sich auf das Verhältnis der Anzahl positiver Ladungen eines Polymers zum Molekulargewicht des Polymers. Die kationische Ladungsdichte multipliziert mit dem Molekulargewicht des Polymers bestimmt die Anzahl der positiv geladenen Stellen auf einer gegebenen Polymerkette. Die Ladungsdichte kann auch definiert werden als die Anzahl der Milliäquivalente der Ladung (quaternärer Stickstoff) pro Gramm (meq/g) des Polymers.

Bei Guar Hydroxypropyltrimonium Chlorid-Verbindungen, die als kationische Guare bezeichnet werden können, kann der Substitutionsgrad, d.h. die Anzahl der durch kationische Gruppen substituierten Hydroxylgruppen an einer bestimmten Zuckereinheit (maximal drei pro Zucker) bestimmt werden. Der Substitutionsgrad wird verwendet, um die N-Äquivalente pro Zuckerteil zu berechnen, was dann zu den Milliäquivalenten der Ladung pro Gramm Polymer führt.

Guar Hydroxypropyltrimonium Chlorid kann beispielsweise von der Firma BASF unter der Handelsbezeichnung Dehyquart Guar N oder unter der Handelsbezeichnung Dehyquart Guar TC bezogen werden.

Wenn in den erfindungsgemäßen Haar-Styling-Zubereitungen wenigstens eine kationisch modifizierte Guar-Verbindung enthalten ist, so liegt diese Verbindung vorteilhaft mit einem Gesamtgehalt von 0,01 Gew.-% bis 2,0 Gew.-%, bevorzugt von 0,05 Gew.-% bis 1,5 Gew.-%, insbesondere bevorzugt von 0,1 Gew.-% bis 1,0 Gew.-% vor, bezogen auf das Gesamtgewicht der Zubereitung und den Aktivgehalt.

In den Haar-Styling-Zubereitungen kann optional wenigstens eine Cellulosemischether-Verbindung enthalten sein. Cellulosemischether können unter den Oberbegriff Celluloseverbindungen eingeordnet werden. Unter Celluloseverbindungen werden alle Polysaccharidverbindungen verstanden, die in ihrer Struktur Verknüpfungen von Glucoseresten enthalten, die über β-1,4-Bindungen verknüpft sind. Neben den unsubstituierten Cellulosen können die Cellulosederivate anionisch, kationisch, amphoter oder nichtionisch sein. Von diesen Derivaten sind die Celluloseether, Celluloseester und Celluloseetherester zu unterscheiden. Unter den Celluloseestern gibt es anorganische Celluloseester (Nitrate, Sulfate oder Phosphate von Cellulose ...), organische Celluloseester (Monoacetate, Triacetate, Amidopropionate, Acetatbutyrate, Acetatpropionate oder Acetattrimellitate von Cellulose ...) und gemischte organische/anorganische Ester von Cellulose, wie Acetatbutyratsulfate und Acetatpropionatsulfate von Cellulose. Von den Celluloseetherestern können die Phthalate von Hydroxypropylmethylcellulose und die Sulfate von Ethylcellulose angegeben werden.

Die erfindungsgemäßen Celluloseverbindungen sind unter den nichtionischen Celluloseethern ausgewählt. Die nichtionischen Celluloseether umfassen Alkylcellulosen, wie beispielsweise Methylcellulosen und Ethylcellulosen; Hydroxyalkylcellulosen, wie beispielsweise Hydroxymethylcellulosen, Hydroxyethylcellulosen und Hydroxypropylcellulosen; gemischte Hydroxyalkyl-alkylcellulosen, die auch als Cellulosemischether bezeichnet werden. *Cellulosemischether* sind Verbindungen wie beispielsweise Hydroxypropylmethylcellulosen, Hydroxyethylmethylcellulosen, Hydroxyethylethylcellulosen und Hydroxybutylmethylcellulosen.

Bevorzugt sind Hydroxypropylmethylcellulosen, geeignete Hydroxypropylmethylcellulosen können beispielsweise unter der Handelsbezeichnung Methocel 40-202 PCG bei der Dow Chemical Comp. bezogen werden.

Hydroxypropylmethylcellulosen können durch die folgende Strukturformel beschrieben werden:

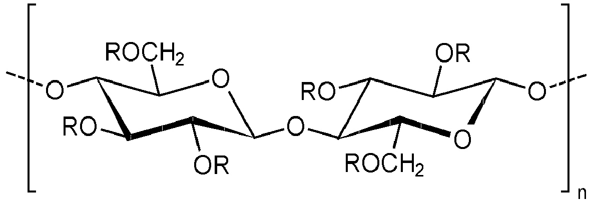

R steht für -H, -CH₃, -CH₂-CHOH-CH₃.

Wenn in den erfindungsgemäßen Haar-Styling-Zubereitungen wenigstens ein Cellulosemischether enthalten ist, so liegt der wenigstens eine Cellulosemischether vorteilhaft mit einem Gesamtgehalt von 0,02 Gew.-% bis 2,0. Gew.-%, bevorzugt von 0,05 Gew.-% bis 1,0 Gew.-%, insbesondere bevorzugt von 0,1 Gew.-% bis 0,5 Gew.-% vor, bezogen auf das Gesamtgewicht der Zubereitung und den Aktivgehalt.

Unter den geeigneten Hydroxypropylmethylcellulosen sind bevorzugt Verbindungen auszuwählen, die eine kalte Verarbeitung, d.h. eine Verarbeitung bei 15 bis 25 °C erlauben.

In den erfindungsgemäßen Haar-Styling-Zubereitungen kann zusätzlich wenigstens ein Moisturizer enthalten sein. Als Moisturizer werden Stoffe oder Stoffgemische bezeichnet, welche kosmetischen Zubereitungen die Eigenschaft verleihen, nach dem Auftragen bzw. Verteilen auf der Haaroberfläche die Feuchtigkeitsabgabe zu reduzieren und/oder die Hydratation der Haaroberfläche bzw. der tiefer liegenden Schichten positiv zu beeinflussen. Vorteilhafte Moisturizer im Sinne der vorliegenden Erfindung sind beispielsweise Glycerin, Sorbitol, Butylenglykol, Propylenglykol, Caprylylglycol, Milchsäure und/oder Lactate, insbesondere Natriumlactat, Biosaccaride Gum-1, Glycine Soja, Ethylhexylglycerin, Pyrrolidoncarbonsäure und Harnstoff. Bevorzugt wird der wenigstens eine Moisturizer aus der Gruppe Glycerin, Sorbitol, Butylenglykol, Propylenglykol, Caprylylglycol, Milchsäure und/oder Salze der Milchsäure ausgewählt. Es können auch zwei oder mehr verschiedene Moisturizer enthalten sind, beispielsweise Sorbitol, Caprylylglycol und Glycerin.

Wenn in den erfindungsgemäßen Haar-Styling-Zubereitungen wenigstens ein Moisturizer enthalten ist, so liegt er mit einem Gesamtgehalt von 0,05 bis 20 Gew. %, bevorzugt 0,15 bis 15,0 Gew. %, bezogen auf das Gesamtgewicht der Zubereitung, vor.

Ausführungsformen der Haar-Styling-Zubereitungen, die sich dadurch auszeichnen, dass sie in Form einer Styling-Emulsion vorliegen, enthalten vorteilhaft wenigstens einen Emulgator, bevorzugt wenigstens zwei Emulgatoren.

Emulsionen benötigen zu ihrer Bildung und zur Stabilisierung im Allgemeinen einen oder mehrere Emulgatoren, gegebenenfalls auch Verdicker und/oder Konsistenzgeber, um über einen kosmetisch akzeptablen Zeitraum stabil zu sein.

Emulgatoren bewirken, dass die zwei nicht miteinander mischbaren Flüssigkeiten (zum Beispiel Öl in Wasser) sich zu einer Emulsion vermengen lassen. Aufgrund des amphiphilen Charakters dringen sie mit ihrem fettlöslichen Teil in das Öl ein. Durch den hydrophilen Teil können die durch Rühren oder Homogenisieren entstandenen Öltröpfchen in der wässrigen Umgebung dispergiert werden. Emulgatoren haben primär keinen waschaktiven, tensidischen Charakter.

Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe).

Vorteilhafte Emulgatoren können aus der Gruppe Trilaureth-4 Phosphate (beispielsweise erhältlich unter Handelsbezeichnung Hostaphat KL 340 D MB bei der Firma Clariant SE), Ceteareth-20 (beispielsweise erhältlich unter Handelsbezeichnung Eumulgin B2 von der Firma BASF), Sodium Stearoyl Glutamate (beispielsweise erhältlich unter Handelsbezeichnung Eumulgin SG von der Firma BASF), Glyceryl Stearate (beispielsweise erhältlich unter Handelsbezeichnung Tegin M Pellets MB von der Firma Evonik), Sodium Cetearyl Sulfate (beispielsweise erhältlich unter Handelsbezeichnung Lanette E granules von der Firma BASF), Glyceryl Stearate SE (beispielsweise erhältlich unter Handelsbezeichnung Tegin VS MB von der Firma Evonik oder unter Handelsbezeichnung Cutina GMS-SE von der Firma BASF), Sorbitanmonostearat (beispielsweise erhältlich unter Handelsbezeichnung SP SPAN 60 MBAL von der Firma Croda GmbH) und/oder Hydrogenated Palm Gylcerides + Potassium Cetyl Phosphate (beispielsweise erhältlich von der Firma Symrise unter der Handelsbezeichnung Emulsiphos) ausgewählt werden.

In der erfindungsgemäßen Emulsion sind bevorzugt die Emulgatoren Trilaureth-4 Phosphat und/oder Sorbinatstearat enthalten

Wenn in der erfindungsgemäßen Haar-Styling-Zubereitung wenigstens ein Emulgator enthalten ist, so liegt dieser wenigstens eine Emulgator mit einem Gesamtgehalt von 0,5 bis 4,0 Gew. %, bevorzugt 1,0 bis 3,0 Gew. % vor, bezogen auf das Gesamtgewicht der Zubereitung.

Es ist ebenso vorteilhaft, wenn in der erfindungsgemäßen Haar-Styling-Zubereitung kein Emulgator im "klassischen Sinne" enthalten ist, wohl aber wenigstens ein kationisches Tensid, das als Emulgator wirkt und die Emulsion stabilisiert.

Dieses kationische Tensid wird bevorzugt aus der Gruppe der Amidoamine. Diese Verbindungen können erhalten werden durch die Reaktion von Fettsäuren mit Diaminen und nachfolgender Quaternierung der freien Aminfunktion. Es ist weiter bevorzugt, wenn das kationische Tensid ausgewählt wird aus der Gruppe Stearylamidopropyltrimmonium Chloride, Ricinoleamidopropyltrimonnium Chloride und/oder Palmitamidopropyltrimonium Chloride; am meisten bevorzugt ist es, wenn das kationische Tensid Palmitamidopropyltrimonium Chlorid ist. Palmitamidopropyltrimonium Chlorid kann als Varisoft PATC bei der Firma Evonik Industries bezogen werden.

Wenn in der erfindungsgemäßen Haar-Styling-Zubereitung wenigstens ein kationisches Tensid enthalten ist, so liegt es mit einem Gehalt von 0,1 bis 4 Gew.-%, bevorzugt 0.2 bis 2 Gew.-% enthalten, bezogen auf das Gesamtgewicht der Zubereitung und bezogen auf den Aktivgehalt.

Ausführungsformen der Haar-Styling-Zubereitungen, die sich dadurch auszeichnen, dass sie in Form eines Haargels vorliegen, enthalten vorteilhaft wenigstens einen natürlichen Verdicker und/oder wenigstens einen Verdicker natürlichen Ursprungs, mit der Maßgabe, dass der natürliche Verdicker nicht Pullulan ist.

Verdicker können auch als "Hydrokolloide" bezeichnet werden, eine technologische Kurzbezeichnung für die an sich richtigere Bezeichnung "hydrophile Kolloide". Hydrokolloide sind Makromoleküle, die eine lineare Gestalt haben können und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit oder Quellbarkeit in Wasser bzw. wässrigen Medien ist. Voraussetzung dafür ist, dass diese Polymere über eine für die Wasserlöslichkeit oder Wasserquellbarkeit ausreichende Anzahl an hydrophilen Gruppen besitzen und nicht zu stark vernetzt sind.

Von bekannten, in der Kosmetik und auch Dermatologie einsetzbaren, Hydrokolloiden werden im Zusammenhang mit dieser Erfindung vorteilhaft folgende Polymere eingesetzt, nämlich:
- organische, natürliche Verbindungen, wie beispielsweise Agar-Agar, Carrageen, Tragant, Gummi arabicum, Alginate/Alginsäure, Pektine, Polyosen, Guar-Mehl, Johannisbrotbaumkernmehl, Dextrine, Gelatine, Casein, Xanthan Gum, Gellan Gum, Succinoglycan Gum, Skleroglucan;
- organische, abgewandelte (modifizierte) Naturstoffe, wie z. B. Carboxymethylcellulose und andere Celluloseether, Hydroxyethyl- und -propylcellulose und dergleichen.

Bevorzugte verdickend wirkende Polymere sind Biopolymere aus verschiedenen Quellen; erfindungsgemäß vorteilhaft kann der wenigstens eine natürliche Verdicker aus der Gruppe Xanthan Gum, Carrageenan und/oder Alginsäure und/oder deren Salzen ausgewählt werden.

Xanthan Gum wird mit Hilfe von Mikroorganismen der Gattung Xanthomonas gewonnen. Xanthan Gum ist ein Polymer, das aus einer Hauptkette aus β-1,4 glykosidisch verknüpften Glucosemolekülen besteht. An jedem zweiten Glucoserest hängt eine Seitenkette aus zwei Mannoseresten, einem Glucoronsäurerest und Pyruvat.

Xanthan Gum löst sich gut in heißem und kaltem Wasser, dabei bilden sich Einfachhelices und Doppelhelices. Es entsteht ein dreidimensionales Netzwerk.

Xanthan Gum kann beispielsweise unter der Handelsbezeichnung Keltrol CG-F V von der Firma CP Kelco bezogen werden.

Carrageenan, auch Carrageen, ist eine Sammelbezeichnung für eine Gruppe langkettiger Kohlenhydrate, die aus Rotalgenzellen gewonnen werden können. Bei Carrageen handelt es sich um lineare, anionische Hydrokolloide, die sich aufgrund ihrer chemischen Struktur unterscheiden lassen und unterschiedliche Eigenschaften aufweisen.

Diese verschiedenen Typen unterscheiden sich durch den Anteil an Galactose und 3,6-Anhydrogalactose sowie über die Anzahl an Sulfatgruppen. Die Sulfatgruppen können in der protonierten Form als freie Säuregruppen vorkommen, oder sie kommen als Salze vor. Dann ist der Verdicker ein Carrageenat. Carrageen ist kommerziell erhältlich.

Carrageenan kann beispielsweise unter der Handelsbezeichnung Gelcarin CP 379 NF von der Firma FMC Health and Nutrition bezogen werden.

Die Salze der Alginsäure (Algin) werden allgemein als Alginate bezeichnet. Alginat ist ein Polysaccharid, das aus den Uronsäuren α-L-Guluronsäure (GulUA) und β-D-Mannuronsäure (ManUA) besteht. Die Uronsäuren sind 1,4-glycosidisch in wechselndem Verhältnis zu linearen Ketten miteinander verbunden. Es bilden sich homopolymere Bereiche, in denen Mannuronsäure oder Guluronsäure als Blöcke vorliegen. Diese Blöcke werden als GG- oder MM-Blöcke bezeichnet. Im Bereich der GG- und MM-Blöcke kommt es zu einer Art Faltstruktur, die bei der Gelierung eine wesentliche Rolle spielt. Insbesondere die GG-Blöcke bilden eine regelmäßige Zickzack-Struktur aus.

Algin kann beispielsweise unter der Handelsbezeichnung Hydagen 558P von der Firma BASF bezogen werden.

Wenn in den erfindungsgemäßen Haar-Styling-Zubereitungen wenigstens ein natürlicher Verdicker und/oder wenigstens ein Verdicker natürlichen Ursprungs enthalten ist. So liegt der wenigstens eine natürliche Verdicker und/oder der wenigstens eine Verdicker natürlichen Ursprungs vorteilhaft mit einem Gesamtgehalt von 0,1 bis 8,0 Gew.-%, bevorzugt 1,0 bis 6,0 Gew.-% vor, bezogen auf das Gesamtgewicht der Zubereitung.

Verbindungen, mit denen der pH-Wert auf einen gewünschten Wert eingestellt werden kann, können vorteilhaft verwendet werden. Geeignet sind alle Verbindungen, die in der kosmetischen Industrie üblich sind, bevorzugt werden jedoch die Verbindungen Citronensäure, Milchsäure und/oder Natronlauge eingesetzt. Der pH-Wert der erfindungsgemäßen Haar-Styling-Zubereitungen liegt für Schaumfestiger vorteilhaft im Bereich 4,8 bis 5,2, für Haargele im Bereich 4,5 bis 6,0 und für Styling-Emulsionen im Bereich von 5,5 bis 6,5.

Erfindungsgemäß vorteilhaft ist ebenfalls die Verwendung einer oder mehrerer Substanzen der Vitamine der B Gruppe und/oder des Vitamin B-Komplexes, die den erfindungsgemäßen Zubereitungen zusätzlich zugesetzt werden können. Substanzen der Vitamin B Gruppe und/oder des Vitamin B-Komplexes sind üblicherweise wasserlöslich und spielen insbesondere für den Zellmetabolismus bei Pflanzen und Tieren eine besondere Rolle.

Beispiele erfindungsgemäßer Substanzen der Vitamine der B Gruppe und/oder des Vitamin B-Komplex sind unter anderem Thiamin (Vitamin B1), Riboflavin (Vitamin B2), Nicotinsäure (Vitamin B3), Nicotinsäureamid (Niacinamid), Pantothensäure (Vitamin B5), Panthenol (Provitamin B5), Panthenoltriacetat, Panthenolmonoethylether, Pantolacton, Pyridoxin und Pyridoxal.

Bevorzugt ist die Verwendung von Panthenol als Substanz der Vitamine der B Gruppe und/oder des Vitamin B-Komplexes in den erfindungsgemäßen Zusammensetzungen.

Panthenol kann beispielsweise unter dem Handelsnamen D-Panthenol 75 W als 77% Lösung in Wasser von der BASF bezogen werden.

Eine oder mehrere Substanzen der Vitamine der B Gruppe und/oder des Vitamin B-Komplex sind erfindungsgemäß in einem Anteil von 0,005 Gew.-% bis 5 Gew.-%, bevorzugt 0,05 Gew.-% bis 2 Gew.-%, insbesondere bevorzugt 0,01 Gew.-% bis 1,0 Gew.-%, bezogen auf das Gesamtgewicht der Haar-Styling-Zubereitung und den Aktivgehalt, in den erfindungsgemäßen Zubereitungen enthalten.

Die erfindungsgemäßen Zubereitungen können konserviert werden. Dazu können alle Konservierungsstoffe verwendet werden, die gemäß Kosmetikverordnung verwendet werden dürfen. Bevorzugt ist jedoch eine Konservierung mit Benzoesäure und/oder ihren Salzen.

Die jeweiligen Konservierungsstoffe können einzeln oder in Kombination eingesetzt werden. Die Gesamtmenge an einem Konservierungsstoff oder mehreren Konservierungsstoffen wird vorteilhaft gewählt aus dem Bereich von 0,01 bis 1,0 Gew.-%, bevorzugt 0,1 bis 0,8 Gew.-%, bezogen auf das Gesamtgewicht der Haar-Styling-Zubereitung und den Aktivgehalt.

Wenn die Haar-Styling-Zubereitungen in Form eines Schaumfestigers vorliegen, sind in der Zubereitung Treibmittel enthalten. Die Treibgase des Treibmittels können aus Kohlenwasserstoffgasen, Dimethylether, Stickstoff, Luft oder Kohlendioxid und/oder Mischungen davon ausgewählt werden. Bevorzugt sind Treibmittel, die eine Mischung aus den Treibgasen Isobutan, Propan und n-Butan sind.

Die bevorzugte Druckgasstufe wird vorteilhaft aus dem Bereich von 2,4 bis 3,5 bar ausgewählt.

Vorteilhaft im Sinne der vorliegenden Erfindung wird der Gewichtsanteil an Treibmittel aus dem Bereich von 15,0 bis 1,0 Gew.-%, insbesondere 12,0 bis 2,0 Gew.-%, bezogen auf das Gesamtgewicht der Gesamtzusammensetzung, die aus Haarschaumfestiger und Treibmittel besteht, gewählt. Der Haarschaumfestiger liegt demnach mit einem Gewichtsanteil von 99 bis 85 Gew.-%, insbesondere 98 bis 88 Gew.-% in der Gesamtzusammensetzung vor.

Die erfindungsgemäßen Haarschaumfestiger liegen vorteilhaft in Form von verschäumbaren oder nachschäumbaren Zusammensetzungen vor, welche aus Aerosolbehältern entnommen werden und beim Austritt aus der Düse aufschäumen. Erfindungsgemäß vorteilhafte Aerosolbehälter sind Sprühvorrichtungen mit einer Füllung aus einem weitgehend flüssigen Haarschaumfestiger, der unter dem Druck eines Treibmittels steht. Derartige Behälter können mit Ventilen sehr unterschiedlicher Bauart ausgestattet sein, die ein Aufschäumen bei der Entnahme des Inhalts ermöglichen.

Um einen Nachweis für einen UV-Schutz von Haaren durch die Kombination von Pullulan und mindestens einer modifizierten Stärke, hier Distarch Phosphate, zu erhalten, wurde eine High Pressure Differential Scanning Calorimetry (HP-DSC) Messung durchgeführt.

Das Säugetier-Haar besteht hauptsächlich aus α-Keratin, das in einer rechtsgängigen α-Helix vorliegt. Das α-Keratin weist eine große Anzahl an hydrophoben Aminosäuren auf (Phenylalanin, Isoleucin, Valin, Methionin und Alanin). Zwei α-Helices des α-Keratins sind umeinander gewunden und bilden eine Super-Helix. Kovalente Quervernetzungen über Disulfid-Brücken stabilisieren diese Superhelix, die auch als Protofilament bezeichnet wird.

Bei einer HP-DSC-Messung durchgeführt mit menschlichem Haar, wird die thermische Stabilität der wichtigsten morphologischen Komponenten des Haares bestimmt. Diese Komponenten können als teilweise helikale, faserige Zwischenfilamente und Proteine, die mit den Zwischenfilament assoziiert sind und eine vernetzte, amorphe Matrix bilden, identifiziert werden. Eine HP-DSC-Messung liefert die Denaturierungs-Temperatur und die Denaturierungs-Enthalpie; der jeweilige Wert hängt vom Ausmaß der strukturellen Integrität des α-helikalen Materials und von der Vernetzungsdichte der Matrix ab.

Eine Schädigung des α-Keratins kann durch äußere Einflüsse, die auf das Haar einwirken, geschehen. Dies kann sowohl Hitze als auch UV-Strahlung sein. Geschädigtes α-Keratin denaturiert schneller, d.h. die Denaturierungstemperatur ist geringer. Wenn nun eine Substanz auf geschädigtes Haar aufgetragen wird und den Denaturierungsprozess verzögert, d.h. die Denaturierungstemperatur liegt höher als bei unbehandeltem geschädigtem Haar, so ist gezeigt, dass diese Substanz bewirkt, dass das α-Keratin und die amorphe Matrix geschützt ist. Dieser Schutz durch die jeweilige Substanz ist nun nicht nur bei einer HP-DSC-Messung wirksam, sondern auch bei äußeren Einflüssen, die auf das Haar einwirken, also auch bei UV-Licht.

Folgende Zubereitungen wurden hergestellt (die Mengenangaben sind in Gew.-% angegeben):

| **INCI** | **Zusammensetzung A** | **Zusammensetzung B** |
|---|---|---|
| Aqua | 93,54 | 96,63 |
| VP/VA Copolymer | 3,00 | - |
| Polyquaternium-68 | 1,00 | - |
| Hydroxypropyl Methylcellulose | - | 0,25 |
| Pullulan | - | 0,75 |
| Distarch Phosphate | - | 1,00 |
| Citric Acid | 0,05 | 0,06 |
| Sodium Hydroxide | - | 0,01 |
| Sodium Benzoate | 0,25 | 0,25 |
| PEG-40 Hydrogenated Castor Oil | 0,25 | - |
| Cetrimonium Chloride | 0,25 | - |
| Palmitamidopropyltrimonium Chloride | - | 0,25 |
| Ceteareth-20 | 0,10 | - |
| Decyl Glucoside | - | 0,25 |
| Benzophenone-4 | 0,05 | - |
| Panthenol | 0,20 | 0,25 |
| Oryzanol | 0,01 | - |
| Niacinamide | 0,05 | 0,05 |
| Parfum | 0,25 | 0,25 |

Die Messung erfolgte an Haarsträhnen (europäisches, ungeschädigtes Haar, jeweils 2 g).

Die Haarsträhnen (Code 01 = undam/untreat, 02 = dam/untreat, 20 = Zusammensetzung A und 40 = Zusammensetzung B) wurden 15 Minuten in Wasser angefeuchtet und anschließend mit einem Beiersdorf-Standard-Haarschampoo (Zusammensetzung siehe Tabelle) gewaschen.

| Inhaltsstoff | [Gew.-%] |
|---|---|
| Laurylethersulfat | 12,5 |
| Natriumchlorid | 1,0 |
| Natriumbenzoat | 0,4 |
| Citronensäure | 0,1 |
| Wasser | 86,0 |

Die Probe mit dem Code 01 für 18 Stunden in einem klimatisierten Raum (22 ±1°C, 55 ±5% relative Luftfeuchtigkeit) getrocknet (unbehandelte und ungeschädigte Probe = =undam/untreat).

Bei der Probe mit dem Code 02 wurde nach dem Waschen das Wasser ausgepresst, dann erfolgte eine UV-Licht-Behandlung für 24 Stunden (Atlas Suntester XLS, 765 W/m, 35°C). Dann wurde die Probe für 18 Stunden in einem klimatisierten Raum (22 ±1°C, 55 ±5% relative Luftfeuchtigkeit) gelagert (unbehandelte und UV-geschädigte Probe = dam/untreat).

Bei der Probe mit dem Code 20 wurde nach dem Waschen das Wasser ausgepresst und dann wurde 0,2g der Zubereitung gemäß Beispielzusammensetzung A vorn und hinten gleichmäßig auf die Haarprobe aufgetragen. Anschließend erfolgte die UV-Behandlung wie für die Probe mit dem Code 02 angegeben. Dann wurde die Probe für 18 Stunden in einem klimatisierten Raum (22 ±1°C, 55 ±5% relative Luftfeuchtigkeit) gelagert (behandelte, UV-geschädigte Probe).

Die Probe mit dem Code 40 wurde auf die gleiche Weise wie die Probe mit dem Code 20 behandelt, aufgetragen wurde in diesem Fall die Zusammensetzung B.

Jede einzelne Haarsträhne wurde zerkleinert (etwa 1 mm lange Stücke) und zwischen 9 mg und 12 mg Haarmaterial in einen Tiegel aus Edelstahl (Large Volume Capsules (LVC), Perkin Elmer Part Number: 0319 0218) überführt. Nach Zugabe von 50 µl demineralisiertem Wasser wurden die Tiegel mit Dichtungsring hermetisch verschlossen und über Nacht bei Raumtemperatur gelagert. Dann erfolgte die HP-DSC-Messung, die für jede Haarsträhne 12mal wiederholt wurde.

Die Messungen erfolgten mit dem Gerät PerkinElmer Pyris 1 DSC, die Software für die Datenanalyse war der Pyris Manager, die Messung erfolgte im Temperaturbereich von 50 - 180°C, die Heizrate betrug 10°C/min.

Folgende Proben (Haarsträhnen) wurden vermessen:

| Probennummer | Art der Probe | Bemerkung |
|---|---|---|
| 01 | Unbehandelte Kontrolle | nicht geschädigt |
| 02 | Unbehandelt | UV geschädigt |
| 20 | Behandelt mit Beispielzusammensetzung A | UV geschädigt |
| 40 | Behandelt mit Beispielzusammensetzung B | UV geschädigt |

Messergebnisse:

| | Peak-Temperatur | |
|---|---|---|
| | Mittelwert | Standardabweichung |
| 01 | 157,18 | 0,57 |
| 02 | 153,70 | 0,22 |
| 20 | 154,48 | 0,26 |
| 40 | 155,37 | 0,34 |

### Beispiele:

Nachfolgende Beispiele sollen die Erfindung verdeutlichen, ohne sie einzuschränken. Dabei geben die angeführten Mengen die Konzentrationen der Rohstoffe an, nicht den Aktivgehalt, jeweils in Gewichtsprozent, sofern keine anderen Angaben gemacht wurden.

### Schaumfestiger:

| **INCI (Aktivgehalt)** | **1** | **2** | **3** | **4** | **5** | **6** | **7** |
|---|---|---|---|---|---|---|---|
| Aqua | 95,80 | 93,59 | 94,40 | 94,69 | 94,80 | 94,90 | 95,00 |
| Hydroxypropyl Methylcellulose | | 0,3 | | 0,3 | | | |
| Distarch Phosphate | 2 | | 1 | | 1 | | |
| Pullulan | 1 | 4 | 2,5 | 2 | 1 | 2 | 1,5 |
| Hydroxypropyl Starch | | | | | 1 | | |
| Corn Starch Modified | | 1 | | 2 | 1 | 2 | 2 |
| Guar Hydroxypropyltrimonium Chloride | | | 1 | | | | 0,5 |
| Citric Acid | 0,1 | 0,1 | 0,1 | 0,1 | 0,1 | 0,1 | 0,1 |
| Sodium Hydroxide | | 0,01 | | 0,01 | | | |
| Sodium Benzoate | 0,25 | 0,25 | 0,25 | 0,25 | 0,25 | 0,25 | 0,25 |
| Panthenol | 0,2 | 0,1 | 0,1 | | 0,2 | 0,1 | |
| PEG-40 Hydrogenated Castor Oil | 0,4 | 0,4 | | 0,4 | | | |
| Decyl Glucoside | | | 0,4 | | | | |
| Caprylyl/Capryl Glucoside | | | | | 0,4 | 0,4 | 0,4 |
| Parfum | 0,25 | 0,25 | 0,25 | 0,25 | 0,25 | 0,25 | 0,25 |

### Styling-Gele:

| **INCI (Aktivgehalt)** | **1** | **2** | **3** | **4** | **5** |
|---|---|---|---|---|---|
| Aqua | 63,30 | 74,11 | 78,90 | 77,94 | 69,5 |
| Hydroxypropyl Methylcellulose | | 0,3 | | 0,3 | |
| Distarch Phosphate | 2 | | 1 | | 2 |
| Pullulan | 1 | 2 | 2 | 3 | 1 |
| Hydroxypropyl Starch | | | | | 1 |
| Corn Starch Modified | | 1 | | 1,5 | 0,5 |
| Guar Hydroxypropyltrimonium Chloride | | | 1 | | |
| Sorbitol | 9,5 | 2 | 3 | 3 | 9,5 |
| Cetearyl Alcohol | | | 5 | 5 | 5 |
| Palmitamidopropyltrimonium Chloride | | | 1 | 1 | |
| PEG-90M | 0,3 | | | | 0,3 |
| Myristyl Alcohol | 5 | 5 | | | |
| Cetyl Alcohol | 5 | 5 | | | |
| Sorbitan Stearate | 2 | 2 | | | 2 |
| Trilaureth-4 Phosphate | 1,8 | 1,5 | | | 2 |
| Ethylhexyl Stearate | | 1 | | | 1 |
| Glycerin | 9 | 5 | 7 | 7 | 5 |
| Panthenol | 0,1 | 0,3 | 0,2 | 0,2 | 0,1 |
| Citric Acid | | 0,05 | | 0,05 | |
| Sodium Hydroxide | | 0,01 | | 0,01 | |
| Hydroxyacetophenone | 0,5 | 0,5 | 0,5 | 0,5 | 0,5 |
| Parfum | 0,5 | 0,5 | 0,4 | 0,5 | 0,6 |

### Styling-Emulsionen:

| **INCI (Aktivgehalt)** | **1** | **2** | **3** | **4** | **5** |
|---|---|---|---|---|---|
| Aqua | 63,30 | 74,11 | 78,90 | 77,94 | 69,5 |
| Hydroxypropyl Methylcellulose | | 0,3 | | 0,3 | |
| Distarch Phosphate | 2 | | 1 | | 2 |
| Pullulan | 1 | 2 | 2 | 3 | 1 |
| Hydroxypropyl Starch | | | | | 1 |
| Corn Starch Modified | | 1 | | 1,5 | 0,5 |
| Guar Hydroxypropyltrimonium Chloride | | | 1 | | |
| Sorbitol | 9,5 | 2 | 3 | 3 | 9,5 |
| Cetearyl Alcohol | | | 5 | 5 | 5 |
| Palmitamidopropyltrimonium Chloride | | | 1 | 1 | |
| PEG-90M | 0,3 | | | | 0,3 |
| Myristyl Alcohol | 5 | 5 | | | |
| Cetyl Alcohol | 5 | 5 | | | |
| Sorbitan Stearate | 2 | 2 | | | 2 |
| Trilaureth-4 Phosphate | 1,8 | 1,5 | | | 2 |
| Ethylhexyl Stearate | | 1 | | | 1 |
| Glycerin | 9 | 5 | 7 | 7 | 5 |
| Panthenol | 0,1 | 0,3 | 0,2 | 0,2 | 0,1 |
| Citric Acid | | 0,05 | | 0,05 | |
| Sodium Hydroxide | | 0,01 | | 0,01 | |
| Hydroxyacetophenone | 0,5 | 0,5 | 0,5 | 0,5 | 0,5 |
| Parfum | 0,5 | 0,5 | 0,4 | 0,5 | 0,6 |

## Patentansprüche

1. Verwendung der Kombination von Pullulan und wenigstens einer modifizierten Stärkeverbindung und/oder wenigstens einer prägelatinisierten Stärke, bevorzugt die Kombination von Pullulan und wenigstens einer modifizierten Stärkeverbindung, in einer Haar-Styling-Zubereitung zum UV-Schutz der Haare, insbesondere der menschlichen Kopfhaare.

2. Verwendung nach Anspruch 1, **dadurch gekennzeichnet, dass** das Gewichtsverhältnis von Pullulan zu der wenigstens einer modifizierten Stärkeverbindung und/oder wenigstens einer prägelatinisierten Stärke1,5:2 bis 1:5, vorzugsweise von 1:2 bis 1:3, beträgt.

3. Verwendung einer Haar-Styling-Zubereitung in Form eines Schaumfestigers, enthaltend die Kombination Pullulan und wenigstens eine modifizierte Stärkeverbindung und/oder wenigstens einer prägelatinisierten Stärke, bevorzugt die Kombination von Pullulan und wenigstens einer modifizierten Stärkeverbindung, zum UV-Schutz der Haare, insbesondere der menschlichen Kopfhaare.

4. Verwendung einer Haar-Styling-Zubereitung in Form eines Haargels, enthaltend die Kombination Pullulan und wenigstens eine modifizierte Stärkeverbindung und/oder wenigstens einer prägelatinisierten Stärke, bevorzugt die Kombination von Pullulan und wenigstens einer modifizierten Stärkeverbindung, zum UV-Schutz der Haare, insbesondere der menschlichen Kopfhaare.

5. Verwendung einer Haar-Styling-Zubereitung in Form einer Styling-Emulsion, enthaltend die Kombination Pullulan und wenigstens eine modifizierte Stärkeverbindung und/oder wenigstens einer prägelatinisierten Stärke, bevorzugt die Kombination von Pullulan und wenigstens einer modifizierten Stärkeverbindung, zum UV-Schutz der Haare, insbesondere der menschlichen Kopfhaare.

6. Verwendung nach Anspruch 3, 4 oder 5, **dadurch gekennzeichnet, dass** der Wassergehalt wenigstens 50 Gew.-%, bevorzugt wenigstens 55 Gew.-%, insbesondere bevorzugt 60 Gew.-% und höchstens 98 Gew.-% beträgt, bezogen auf das Gesamtgewicht der Zubereitung.

7. Verwendung nach Anspruch 3, 4 oder 5 und/oder Anspruch 6, **dadurch gekennzeichnet, dass** Pullulan mit einem Gehalt von 0,1 bis 15,0 Gew.-%, bevorzugt von 0,5 bis 5,0 Gew.-% enthalten, bezogen auf das Gesamtgewicht der Zubereitung.

8. Verwendung nach Anspruch 3, 4 oder 5 und/oder Anspruch 6 und/oder 7, **dadurch gekennzeichnet, dass** die wenigstens eine modifizierte Stärkeverbindung ausgewählt wird aus der Gruppe Corn Starch Modified, Hydrolyzed Corn Starch, Distarch Phosphate und/oder Hydroxyproyl Starch Phosphate, bevorzugt Distarch Phosphate.

9. Verwendung nach Anspruch 3, 4 oder 5 und/oder wenigstens einem der Ansprüche 6 bis 8, **dadurch gekennzeichnet, dass** die wenigstens eine modifizierte Stärkeverbindung mit einem Gesamtgehalt von 0,1 bis 8,0 Gew. %, bevorzugt von 0,5 bis 5,0 Gew.-% vorliegt, bezogen auf das Gesamtgewicht der Zubereitung.

10. Verwendung nach Anspruch 3, 4 oder 5 und/oder wenigstens einem der Ansprüche 6 bis 9, **dadurch gekennzeichnet, dass** die wenigstens eine prägelatinisierte Stärke mit einem Gesamtgehalt von 0,1 bis 8,0 Gew. %, bevorzugt von 0,5 bis 5,0 Gew. % vorliegt, bezogen auf das Gesamtgewicht der Zubereitung.

11. Verwendung nach Anspruch 3, 4 oder 5 und/oder wenigstens einem der Ansprüche 6 bis 10, **dadurch gekennzeichnet, dass** zusätzlich wenigstens eine weitere Film- bildende Verbindung ausgewählt aus kationisch modifizierten Guar-Verbindungen, bevorzugt Guar Hydroxypropyltrimonium Chlorid enthalten ist.

12. Verwendung nach Anspruch 3, 4 oder 5 und/oder wenigstens einem der Ansprüche 6 bis 11, **dadurch gekennzeichnet, dass** die wenigstens eine kationisch modifizierte Guar-Verbindung mit einem Gesamtgehalt von 0,01 Gew.-% bis 2,0 Gew.-%, bevorzugt von 0,05 Gew.-% bis 1,5 Gew.-%, insbesondere bevorzugt von 0,1 Gew.-% bis 1,0 Gew.-% enthalten ist, bezogen auf das Gesamtgewicht der Zubereitung und den Aktivgehalt.

13. Verwendung nach Anspruch 3, 4 oder 5 und/oder wenigstens einem der Ansprüche 6 bis 12, **dadurch gekennzeichnet, dass** zusätzlich wenigstens ein Cellulosemischether, bevorzugt eine Hydroxypropylmethylcellulose enthalten ist.

14. Verwendung nach Anspruch 3, 4 oder 5 und/oder wenigstens einem der Ansprüche 6 bis 13, **dadurch gekennzeichnet, dass** der wenigstens eine Cellulosemischether mit einem Gesamtgehalt von 0,02 Gew.-% bis 2,0. Gew.-%, bevorzugt von 0,05 Gew.-% bis 1,0 Gew.-%, insbesondere bevorzugt von 0,1 Gew.-% bis 0,5 Gew.-% enthalten ist, bezogen auf das Gesamtgewicht der Zubereitung und den Aktivgehalt.

15. Verwendung nach Anspruch 3 und/oder wenigstens einem der Ansprüche 6 bis 14, **dadurch gekennzeichnet, dass** ein Treibmittel, ausgewählt aus der Gruppe Kohlenwasserstoffgase, Dimethylether, Stickstoff, Luft, Kohlendioxid oder geeignete Mischungen davon, bevorzugt eine Mischung Isobutan, Propan und n-Butan, enthalten ist.

16. Verwendung nach Anspruch 3 und/oder wenigstens einem der Ansprüche 6 bis 15, **dadurch gekennzeichnet, dass** Treibmittel mit einem Gehalt von 15,0 bis 1,0 Gew.-%, insbesondere 12,0 bis 2,0 Gew.-% enthalten ist, bezogen auf das Gesamtgewicht der Gesamtzusammensetzung.
